(19) Europäisches Patentamt
European Patent Office
Office européen des brevets

(11) Publication number: 0 007 240
B1

(12) **EUROPEAN PATENT SPECIFICATION**

(45) Date of publication of patent specification: 15.02.84

(51) Int. Cl.³: C 07 D 307/93

(21) Application number: 79301397.0

(22) Date of filing: 13.07.79

(54) **Process for dehydroxylation of 13-hydroxygibberellins and intermediates useful in the process.**

(30) Priority: 18.07.78 GB 3017378
27.11.78 US 963743

(43) Date of publication of application:
23.01.80 Bulletin 80/2

(45) Publication of the grant of the patent:
15.02.84 Bulletin 84/7

(84) Designated Contracting States:
AT BE CH DE FR GB IT LU NL SE

(56) References cited:
GB - A - 892 777

JOURNAL OF THE CHEMICAL SOCIETY PERKIN TRANSACTIONS I, no. 9, 1976, London L.J. BEELEY et al.: "Partial synthesis of 2-hydroxygibberellins;Characterisation of two new gibberellins A46 and A47", pages 1022-28.

The file contains technical information submitted after the application was filed and not included in this specification

(73) Proprietor: IMPERIAL CHEMICAL INDUSTRIES PLC
Imperial Chemical House Millbank
London SW1P 3JF (GB)

(72) Inventor: Beale, Michael Henry Dept. of Chemistry
University of British Columbia
Vancouver 8 (CA)
Inventor: MacMillan, Jake School of Chemistry
University of Bristol
Bristol BS8 1TS (GB)

(74) Representative: Fawcett, Richard Fennelly et al,
Imperial Chemical Industries PLC Legal
Department: Patents PO Box 6
Welwyn Garden City Herts, AL7 1HD (GB)

(56) References cited:
AGRICULTURAL AND BIOLOGICAL CHEMISTRY, vol. 41, no. 6, 1977, Japan. N.MUROFUSHI et al.: "Preparation of radioactive gibberellins A20, A5 and A8", pages 1075-79

JOURNAL OF CHEMICAL SOCIETY, part III, 1965, no. 652 London B.E. CROSS et al.: "Gibberellic acid. Part XXX. The preparation of 7-deoxygibberellins from gibberellic acid", pages 3555-63

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 007 240 B1

Courier Press, Leamington Spa, England.

Process for dehydroxylation of 13-hydroxygibberellins and intermediates useful in the process

This invention relates to a process for the dehydroxylation of 13-hydroxygibberellins and to new intermediates prepared during the process.

Gibberellins are a well-recognised group of tetracyclic compounds which have been found to be of use as plant growth hormones. Many gibberellins are found to occur naturally in plants and some gibberellins can be produced by fermentation methods. Over fifty gibberellins have now been reported in the literature. Gibberellins are based on the tetracyclic ring structure of formula I which shows the ring numbering (but not the precise stereochemistry).

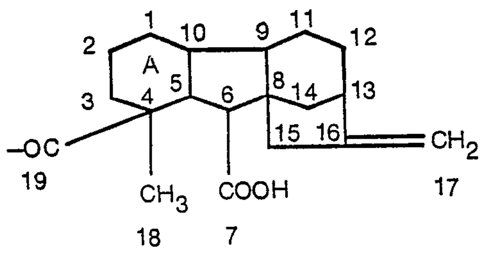

I

One gibberellin which can be readily obtained by fermentation methods is the gibberellin known as $GA_3$ of formula II

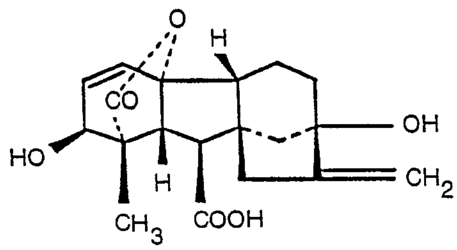

II

$GA_3$, like many other gibberellins has a 13-hydroxy group. This hydroxy group is a tertiary hydroxy group located at one of the bridge heads in the molecule. In the gibberellin field, it is often found that removal of a 13-hydroxy group, or replacement of the 13-hydroxy group by another substituent can have advantageous effects on the overall properties of the compound, for example, the 13-deoxy analogue of $GA_3$ which is $GA_7$, is known to exhibit advantageous properties not shown by $GA_3$.

Consequently, it would be advantageous to have available a process for the 13-dehydroxylation of 13-hydroxygibberellins so that they could be converted, semi-synthetically, into gibberellins which are either unsubstituted at the 13-position or substituted by another group.

Conventional methods of dehydroxylating organic compounds, e.g. steroids, are not satisfactory for use with gibberellins because of the other sensitive sites in the gibberellin molecule which would be adversely affected during the dehydroxylation reaction. For example, many gibberellins have one or more further hydroxy groups in the molecule which it is desired to retain and which would be removed together with the 13-hydroxy group during the relatively severe reaction conditions that are necessary to remove this tertiary hydroxy group [Cross *et al*, JCS, III, p. 3555—63 and Beeley *et al*, JCS, I. p. 1022—8]. $GA_3$, mentioned above, is one such gibberellin and in addition $GA_3$ includes a 19,10-lactone bridge which is also sensitive to many reaction conditions, tending to migrate easily to the 2,4-position.

It is also necessary to select dehydroxylation conditions which will not adversely affect the 7-carboxy group or the 17-methylene group present in all gibberellins.

We have now found that 13-dehydroxylation of 13-hydroxygibberellins can be achieved satisfactorily by converting the 13-hydroxy group in a 13-hydroxygibberellin into one of certain esters and reducing the resulting ester with an organotin hydride or organosilane.

The invention therefore provides a process for the 13-dehydroxylation of a 13-hydroxygibberellin, the 7-carboxy group of the 13-hydroxygibberellin being optionally protected (e.g. in the form of an unsubstituted or halo-, nitro-, alkyl-, alkoxy- or trifluoromethyl-substituted phenacyl ester or a silyl ester) and the 13-hydroxygibberellin containing an allylic hydroxy group and 19,10-lactone bridge, characterised by converting the 13-hydroxy group (optionally in the form of an alkali metal salt) in the 13-hydroxygibberellin to a phosphate, sulphonate, carboxylate (including haloformate for example chloroformate), thiocarboxylate or dithiocarbonate ester (for example by reaction with an esterifying agent which is a phosphoric acid, a sulphonic acid or a carboxylic acid or an esterifying derivative of one of said acids) and then reducing the 13-ester so formed in the presence of a free-radical initiator and an organotin hydride or organosilane to reduce the 13-ester and form a 13-dehydroxylated gibberellin, which if necessary is reacted in known manner to convert the 7-protecting group to a 7-protecting group to a 7-carboxy group.

The esterifying agent for use in forming the 13-ester is suitably an (unsubstituted or halo-substituted alkane)sulphonyl halide, an (unsubstituted or halo-, nitro-, alkyl-, alkoxy- or trifluoromethyl-substituted arane)sulphonyl halide or an (unsubstituted or halo-, nitro-, alkyl-, alkoxy- or trifluoromethyl-substituted arane)carboxylic acid halide. It is preferably an organosulphonyl halide. Examples of preferred esterifying agents are an unsubstituted alkanesulphonyl chloride, for example methane- or ethane-sulphonyl chloride, an unsubstituted or alkyl-substituted aranesulphonyl chloride, for example p-toluenesulphonyl or benzenesulphonyl chloride, or an unsubstituted aranecarboxylic acid chloride for example benzoic acid chloride. Another possible esterifying agent is a trifluoroacetyl or trifluoromethanesulphonyl halide (e.g. chloride). In addition to being in the form of a free acid or an acid halide, the esterifying agent can be in the form of an anhydride or ester. When the esterification is carried out using the acid halide, it will normally be carried out in the presence of a hydrogen halide acceptor, typically a secondary or tertiary amine such as triethylamine, pyridine or piperidine and using a solvent e.g. toluene, methylene chloride or pyridine. To optimise the reaction, the esterification is normally carried out with equimolar amounts of the acid halide and of the hydrogen halide acceptor.

During the esterification of the 13-hydroxy group with the acid halide it is possible that the 7-carboxy group will be converted to the acid halide. This conversion can be monitored by the acidity deriving from the presence of the free carboxy group in for example the sulphonated product and, before proceeding to the next step of the process, it is usually desirable to reinstate the 7-carboxy group by, for example, treatment with sodium carbonate or sodium bicarbonate in aqueous acetone.

The thiocarboxylate and dithiocarbonate esters of the 13-hydroxygibberellin can be prepared in known manner. For example the thiocarboxylate esters can be prepared by reacting the 13-hydroxygibberellin with an imidoyl chloride methochloride (prepared from phosgene and a tertiary amide) and reacting the salt so obtained with hydrogen sulphide in pyridine, while the dithiocarbonate esters can be prepared by reacting the 13-hydroxygibberellin with carbon disulphide in the presence of sodium hydride and imidazole and alkylating the salt so obtained.

The haloformate esters can also be prepared in known manner; for example the chloroformate esters can be prepared by heating the 13-hydroxygibberellin with phosgene.

The final step in the process involves the reduction of the 13-ester group with an organotin hydride or organosilane. The organic reagent is suitably one having the general formula

$$R_xMH_y$$

wherein M is Sn or Si, R is unsubstituted alkyl, aryl or aralkyl; x and y are 1, 2, or 3, the sum of x and y being 4; and, when x is 2 or 3, the groups R being the same or different. Preferably, R is $C_{1-6}$ alkyl (e.g. n-butyl), phenyl or benzyl, x is 3 and y is 1. The reagent is preferably used in the freshly prepared state (and in fact can be formed *in situ*), and can be prepared in known manner. For example the trialkyltin hydrides can be prepared by treating the trialkyltin chloride with lithium aluminium hydride or by treating a bis(trialkyltin) oxide with polymethylhydrosiloxane; these reagents have been found to react in a specific manner with the 13-sulphonyloxy ester group in the gibberellins to remove the 13-ester.

Availability points to the use of tri-n-butyltin hydride or tri-n-propylsilane as the reagent of choice.

The organotin hydride and organosilane reagents probably function by a free radical mechanism and it is therefore advantageous to have present in the reaction mixture a source of free radicals. For this purpose, we have found $\alpha$-azo-isobutyronitrile to be suitable. Alternatively, it may be possible to generate free radicals using radiation.

Under our experimental conditions, we have found that it is possible to bring about substantially complete conversion of the 13-sulphonyloxy ester group in the gibberellins to the 13-unsubstituted compound by heating the reaction mixture in an organic diluent such as benzene, toluene or other solvent for up to 48 hours.

As mentioned above, one of the main advantages of the present process is its selectivity to the 13-dehydroxylation of gibberellins containing sensitive substituents in other parts of the molecule. Where the gibberellin contains potentially susceptible substituents in other parts of the molecule, for example, other hydroxy groups which it is desired to retain, it is desirable to protect these other hydroxy groups during the 13-dehydroxylation. This can be done by conventional methods and we have found in the $GA_3$ series, for example, that the 3-hydroxy group can be satisfactorily protected by acylation e.g. acetylation. However acetylation is only one method of protecting the hydroxy group and other protecting groups (e.g. ether groups or ester groups), conventionally used in gibberellin or steroid chemistry can be used.

The advantage of using acylation in the $GA_3$ series is that the acyl group can be removed after the 13-dehydroxylation by treating the 13-dehydroxylated product with an alcoholic alkali, for example sodium or potassium carbonate, the avoidance of an aqueous system thereby ensuring that the lactone bridge is not opened or isomerised.

The present process is particularly applicable to gibberellins containing additional hydroxy groups in the molecule which it is desired to retain. These additional hydroxy groups will normally be secondary hydroxy groups and our dehydroxylation is of particular value with those gibberellins containing allylic secondary hydroxy groups such as in the $GA_3$ series.

Our process is particularly useful for the conversion of $GA_3$ to $GA_7$ since it is possible to protect the 3-hydroxy group initially by acetylation, to esterify the 13-hydroxy group, preferably with methylsulphonyl chloride to form 3-acetyl-13-mesyloxy-$GA_3$ 17-acid chloride, to reconvert the acid chloride group back to the carboxy group, to split off the mesyloxy group by treatment of the ester with tri-n-butyltin hydride or tri-n-propylsaline and finally to remove the acetyl protecting group at the 3-position. In this sequence of reactions, each step can be carried out in a selective manner so that, for example, esterification of the 3-hydroxy group and then the 13-hydroxy groups followed by demesylation and removal of the 3-hydroxy protecting group can all be carried out under conditions which do not open or isomerise the lactone ring.

This reaction has been developed primarily for the conversion of $GA_3$ to $GA_7$, since, although $GA_7$ can be prepared directly by fermentation, it is always produced in association with $GA_4$ (its analogue having a saturated A ring) and it is difficult to separate the mixture of $GA_7$ and $GA_4$. $GA_3$ can be readily produced by fermentation and our process provides a selective semi-synthetic route for producing $GA_7$ free from $GA_4$.

As indicating above, the process can be performed on a 13-hydroxygibberellin wherein the 7-carboxy group is protected (e.g. in the form of an unsubstituted or substituted phenacyl ester or a silyl ester). The protecting group is a group which can be removed without adversely affecting the rest of the gibberllins molecule. A suitable phenacyl ester is the p-methoxyphenacyl ester. The silyl ester is suitably a trialkylsilyl ester e.g. a dimethyl-t-butylsilyl ester. These esters can be removed in known manner to give the free acid without adversely affecting the rest of the gibberellin molecule. The silyl ester can be removed by reaction with fluoride ion (e.g. sodium or potassium fluoride). The phenacyl ester group can be removed using zinc and acetic acid or photolysis ($\lambda \geqslant$ 300 mm) in ethanolic solution (see Bateson and Cross, JCS, 1974, 2409, the disclosure of which document is incorporated herein by reference).

The phosphate, sulphonate, carboxylate, thiocarboxylate and dithiocarbonate esters of the 13-hydroxygibberellins, for example the 13-organophosphoryloxy, 13-organosulphonyloxy, 13-organocarbonyloxy, 13-organothiocarbonyloxy or 13-organodithiocarbonyloxy derivatives of a 13-hydroxygibberellin (for example $GA_3$), the 13-hydroxygibberellin containing an allycyclic hydroxy group in ring A and a 19,10-lactone bridge and any secondary hydroxy groups and the 7-carboxy groups in the gibberellin molecule being optionally protected, produced as intermediates in the process of the invention are new compounds. Suitable protecting groups for the 7-carboxy groups are discussed above. The secondary hydroxy groups may be protected by any of the protecting groups customarily used in gibberellin or steroid chemistry, for example as a removable ether or ester group. It is preferred that such protecting group be organocarbonyl groups such as $C_{1-4}$ alkanoyloxy groups or aroyl gorups, e.g. benzoyl. When the hydroxy group is etherified, it may be etherified for example as a tetrahydropyranyl ether or as a silyl ether.

The present invention provides a new derivative of $GA_3$ of the formula

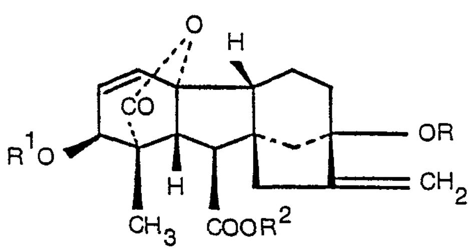

wherein R is (unsubstituted or halo-substituted alkane)sulphonyl, (unsubstituted or halo-, nitro-, alkyl-, alkoxy-, or trifluoromethyl-substituted arane)sulphonyl or (unsubstituted or halo-, nitro-, alkyl-, alkoxy-, or trifluoromethyl-substituted arane)carbonyl or thiocarbonyl, $R^1$ is hydrogen or acyl (preferably alkanoyl e.g. acetyl), and $R^2$ is hydrogen, silyl or unsubstituted or halo-, nitro-, alkyl-, alkoxy-, or trifluoromethyl-substituted phenacyl.

The corresponding acid chlorides, which can be obtained during esterification of the 13-hydroxygibberellin with an acid chloride, are also new compounds and form part of this invention.

Our studies have been carried out on naturally occurring gibberellins or gibberellins obtained by fermentation. In all these compounds, the substituents at the 3-, 4- and 6-positions are in the $\beta$-configuration and the configuration of these substituents is retained after the dehydroxylation process of the invention. Consequently, the dehydroxylation process can be carried out without influencing the configuration of the gibberellins.

The following Example is given to illustrate the invention.

Example

This Example describes the conversion of $GA_3$ to $GA_7$ by protecting the 3-hydroxy group by acetylation, mesylating the 13-hydroxy group with mesyl chloride, reconverting the acid chloride group to the carboxylic acid group, removing the mesyloxy group by reaction with tri-n-butyltin hydride and

finally removing the protecting acetoxy group at the 3-position by treatment with methanolic potassium carbonate.

a) 3-Acetyl-$GA_3$

$GA_3$ (1 g) was treated with 6.4 ml of acetic anhydride in pyridine (12 ml) overnight. The reaction mixture was poured into water, acidified and extracted with ethyl acetate. The product, obtained on removal of the ethyl acetate, was fractionally crystallised from acetone/petroleum ether to give 825 mg of pure 3-acetyl-$GA_3$.

b) 3-Acetyl-13-Mesyloxy-$GA_3$

The crystalline product from step a) (825 mg) was dissolved in 10 ml pyridine and 1 ml triethylamine and 1 ml methanesulphonyl chloride was added. The mixture was stirred for 5 hours and then poured into water. The aqueous mixture was acidified and extracted with ethyl acetate. At this stage the product was found to consist mainly of 3-acetyl-13-mesyloxy-$GA_3$ in the carboxylic acid chloride form. The product was dissolved in 40 ml acetone to which 20 ml saturated sodium hydrogen carbonate and 20 ml water was added. The mixture was stirred for 15 hours at about 20°C and then the acetone removed *in vacuo*. The aqueous residue was then acidified with hydrochloric acid and extracted with ethyl acetate. The organic residue was then absorbed on to silica and chromatographed on a silica column (120 g) (30 × 3 cm) made up in petroleum ether.

The column was then eluted initially with petroleum ether and a 250 ml fraction collected. The column was then eluted with successive 250 ml fractions of petroleum ether containing increasing quantities of ethyl acetate as follows:

| Proportion of ethyl acetate in percent by volume | Fraction Number |
|---|---|
| 0 | 1 |
| 5 | 2 |
| 10 | 3 |
| 20 | 4 |
| 100 | 5,6 |

Fractions 1—4 were rejected. Fractions 5 and 6 were found to contain 3-acetyl-13-mesyloxy-$GA_3$ (901 mg).

The identity of the product was confirmed by mass spectrometry and NMR, NMR giving a 3 proton singlet peak at $\delta 3$ confirming the presence of the 13-methanesulphonyl group and another 3 proton sinlget at $\delta 2.05$ indicating the presence of the 3-acetoxy group. Corresponding peaks were also found for the carboxylic acid chloride analogue of this compound which was also examined by NMR.

c) 3-Acetyl-13-Deoxy-$GA_3$

The diester recovered in fractions 5 and 6 above (901 mg) was dissolved in 25 ml benzene and 20 mg $\alpha$-azoisobutyronitrile and 2 ml of freshly prepared tri-n-butyltin hydride added. The mixture was refluxed under nitrogen for 24 hours after which time it was found that approximately 60% of the gibberellin had been dehydroxylated. A further 500 $\mu$l of tri-n-butyltin hydride and 10 mg of $\alpha$-azo-isobutyronitrile were then added and the mixture heated again under nitrogen for a further 15 hours.

TLC examination after this second period of heating indicated that the reaction was now complete. The benzene was removed *in vacuo* and the product absorbed, from an acetone solution, on to silica and chromatographed on a column of silica (120 g, 30 × 3 cm) made up in petroleum ether. The column was eluted with petroleum ether containing increasing quantities of ethyl acetate — 250 ml fractions were collected as follows:

| Proportion of ethyl acetate in percent by volume | Fraction Number |
|---|---|
| 0 | 1 |
| 5 | 2 |
| 10 | 3 |
| 100 | 4,5 |

Fractions 1—3 contained residues from tri-n-butyltin hydride and were discarded. Fractions 4 and 5 yielded an oil (850 mg) containing 3-acetyl-13-deoxy-$GA_3$ together with some tin containing residues.

d) $GA_7$

The product from step c) above (850 mg) was taken up in 20 ml methanol and 425 mg potassium carbonate were added. This mixture was stirred at about 20°C for 15 hours and then 4 g of an ion exchange resion in the acid form (Amberlite IR-120, $H^+$) was added to remove the potassium carbonate. After 15 minutes this mixture was filtered and the organic solvent removed. The residue was partitioned between ethyl acetate and sodium hydrogen carbonate solution.' $GA_7$ (419 mg) was recovered from the bicarbonate phase and shown to be identical (TLC and NMR) to an authentic sample.

**Claims**

1. A process for the 13-dehydroxylation of a 13-hydroxygibberellin, the 7-carboxy group of the 13-hydroxygibberellin being optionally protected and the 13-hydroxygibberellin containing an allylic hydroxy group and a 19,10-lactone bridge, characterised by converting the 13-hydroxy group, optionally in the form of an alkali metal salt, in the 13-hydroxygibberellin to a phosphate, sulphonate, carboxylate, thiocarboxylate or dithiocarbonate ester, and then reducing the 13-ester so formed in the presence of a free-radical initiator and of an organotin hydride or organosilane to reduce the 13-ester and form a 13-dehydroxylated gibberellin, which if necessary is reacted in known manner to convert the 7-protecting group to a 7-carboxy group.

2. A process according to claim 1 characterised in that the 13-ester is formed by reacting the 13-hydroxygibberellin with an esterifying agent which is a phosphoric acid, a sulphonic acid or a carboxylic acid or an esterifying derivative of one of said acids, for example an (unsubstituted or halo-substituted alkane)sulphonyl halide, an unsubstituted or halo-, nitro-, alkyl-, alkoxy- or trifluoromethyl-substituted arene)sulphonyl halide or an unsubstituted or halo-, nitro-, alkyl-, alkoxy- or trifluoromethyl-substituted arene)-carboxylic acid halide.

3. A process according to claim 2 characterised in that the esterifying agent is an unsubstituted alkane-sulphonyl chloride, for example methane- or ethane-sulphonyl chloride, an unsubstituted or alkyl-substituted arene-sulphonyl chloride, for example p-toluenesulphonyl or benzenesulphonyl chloride, or an unsubstituted arene-carboxylic acid chloride, for example benzoic acid chloride.

4. A process according to any one of claims 1 to 3 characterised in that the organotin hydride or organosilane has the formula

$$R_xMH_y$$

wherein M is Sn or Si, R is unsubstituted alkyl, aryl or aralkyl and x and y are 1, 2 or 3, the sum of x and y being 4; and, when x is 2 or 3, the groups R being the same or different.

5. A process according to claim 4 characterised in that, in the organotin hydride or organosilane, R is $C_{1-6}$ alkyl, phenyl or benzyl, x is 3 and y is 1.

6. A process according to claim 5 characterised in that the organotin hydride is tri-n-butyltin hydride or the organosilane is tri-n-propylsilane.

7. A process according to any one of claims 1 to 6 characterised in that the 13-hydroxygibberellin contains at least one further hydroxy group.

8. A process according to claim 7 characterised in that the further hydroxy group is protected before esterification of the 13-hydroxy group and the protecting group removed before, after and/or during the 13-dehydroxylation.

9. A process according to any one of claims 1 to 8 characterised in that the 7-protected group in the 13-hydroxygibberellin is an unsubstituted or halo-, nitro-, alkyl-, alkoxy- or trifluoromethyl-substituted phenacyl ester or a silyl ester.

10. A process according to claim 9 characterised in that the 13-hydroxygibberellin is $GA_3$ and the 13-dehydroxylated gibberellin is $GA_7$.

11. A process according to claim 10 characterised in that $GA_3$ is acetylated to form 3-acetyl-$GA_3$, the 3-acetyl-$GA_3$ is treated with mesyl chloride to form 3-acetyl-13-mesyloxy-$GA_3$, the 3-acetyl-13-mesyloxy-$GA_3$ is treated with tri-n-butyltin hydride or tri-n-propylsilane in the presence of $\alpha$-azo-isobutyronitrile and an inert organic diluent to form 3-acetyl-13-deoxy-$GA_3$, and the 3-acetyl-13-deoxy-$GA_3$ is treated with alkali to deacylate the 3$\beta$-acetyl-13-deoxy-$GA_3$ to form $GA_7$.

12. A compound of the formula:

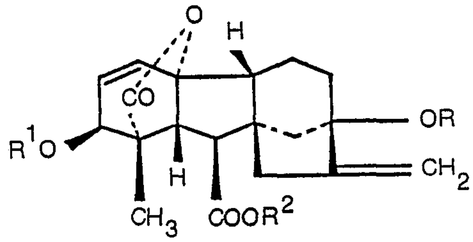

where R is (unsubstituted or halo-substituted alkane)sulphonyl, (unsubstituted or halo-, alkyl- or trifluoromethyl-substituted benzene)sulphonyl or (unsubstituted or halo-, alkyl- or trifluoromethyl-substituted benzene)carbonyl or thiocarbonyl, $R^1$ is hydrogen or acyl (preferably alkanoyl e.g. acetyl), and $R^2$ is hydrogen, silyl or unsubstituted or halo-, nitro-, alkyl-, alkoxy-, or trifluoromethyl-substituted phenacyl.

13. A compound according to claim 12 characterised in that R is methanesulphonyl, p-toluenesulphonyl, benzenesulphonyl or benzoyl.

14. 3-Acetyl-13-mesyloxy-$GA_3$.

15. A compound according to claim 12 in the form of an acid halide (e.g. acid chloride).

**Patentansprüche**

1. Verfahren zur 13-Dehydroxylierung eines 13-Hydroxygibberellins, wobei die 7-Carboxygruppe des 13-Hydroxygibberellins gegebenenfalls geschützt ist und das 13-Hydroxygibberellin eine allylische Hydroxygruppe und eine 19,10-Lactonbrücke aufweist, dadurch gekennzeichnet, daß die gegebenfalls in Form eines Alkalimetallsalzes vorliegende 13-Hydroxygruppe im 13-Hydroxygibberellin in einen Phosphat-, Sulfonat-, Carboxylat-, Thiocarboxylat- oder Dithiocarbonatester überführt wird und hierauf der so gebildete 13-Ester in Gegenwart eines radikalischen Initiators und eines Organozinnhydrids oder Organosilans reduziert wird, um den 13-Ester zu reduzieren und ein 13-dehydroxyliertes Gibberellin zu bilden, welches nötigenfalls in bekannter Weise umgesetzt wird, um die 7-Schutzgruppe in eine 7-Carboxygruppe umzuwandeln.

2. Verfahren nach Anspruch 1, dadurch gekennzeichnet, daß der 13-Ester dadurch gebildet wird, daß das 13-Hydroxygibberellin mit einem Veresterungsmittel umgesetzt wird, welches eine Phosphorsäure, eine Sulfonsäure oder eine Carbonsäure oder ein veresterndes Derivat einer dieser Säuren ist, wie z.B. ein unsubstituiertes oder halogen-substituiertes Alkansulfonylhalogenid, ein unsubstituiertes oder halogen-, nitro-, alkyl-, alkoxy- oder trifluoromethyl-substituiertes Arensulfonylhalogenid oder ein unsubstituiertes oder halogen-, nitro-, alkyl-, alkoxy- oder trifluoromethyl-substituiertes Arencarbonsäurehalogenid.

3. Verfahren nach Anspruch 2, dadurch gekennzeichnet, daß das Veresterungsmittel ein unsubstituiertes Alkansulfonylchlorid, wie z.B. Methan- oder Ethansulfonylchlorid, ein unsubstituiertes oder alkylsubstituiertes Arensulfonylchlorid, wie z.B. p-Toluolsulfonyl- oder Benzolsulfonylchlorid, oder ein unsubstituiertes Arencarbonsäurechlorid, wie z.B. Benzoesäurechlorid, ist.

4. Verfahren nach einem der Ansprüche 1 bis 3, dadurch gekennzeichnet, daß das Organozinnhydrid oder Organosilan die Formel

$$R_xMH_y$$

aufweist, worin M für Sn oder Si steht, R für unsubstituiertes Alkyl, Aryl oder Aralkyl steht und x und y für 1, 2 oder 3 stehen, wobei die Summe aus x und y 4 ist und, wenn x für 2 oder 3 steht, die Gruppen R gleich oder verschieden sind.

5. Verfahren nach Anspruch 4, dadurch gekennzeichnet, daß im Organozinnhydrid oder Organosilan R für $C_{1-6}$-Alkyl, Phenyl oder Benzyl, x für 3 und y für 1 steht.

6. Verfahren nach Anspruch 5, dadurch gekennzeichnet, daß das Organozinnhydrid Tri-n-butylzinnhydrid oder das Organosilan Tri-n-propylsilan ist.

7. Verfahren nach einem der Ansprüche 1 bis 6, dadurch gekennzeichnet, daß das 13-Hydroxygibberellin mindestens eine weitere Hydroxygruppe enthält.

8. Verfahren nach Anspruch 7, dadurch gekennzeichnet, daß die weitere Hydroxygruppe vor der Veresterung der 13-Hydroxygruppe geschützt wird und die Schutzgruppe vor, nach und/oder während der 13-Dehydroxylierung entfernt wird.

9. Verfahren nach einem der Ansprüche 1 bis 8, dadurch gekennzeichnet, daß die geschützte 7-Gruppe im 13-Hydroxygibberellin ein unsubstituierter oder halogen-, nitro-, alkyl-, alkoxy- oder trifluoromethyl-substituierter Phenacylester oder ein Silylester ist.

10. Verfahren nach Anspruch 9, dadurch gekennzeichnet, daß das 13-Hydroxygibberellin $GA_3$ und das 13-dehydroxylierte Gibberellin $GA_7$ ist.

11. Verfahren nach Anspruch 10, dadurch gekennzeichnet, daß $GA_3$ acetyliert wird, um 3-Acetyl-

$GA_3$ zu bilden, das 3-Acetyl-$GA_3$ mit Mesylchlorid behandelt wird, um 3-Acetyl-13-mesyloxy-$GA_3$ zu bilden, das 3-Acetyl-13-mesyloxy-$GA_3$ in Gegenwart von $\alpha$-Azo-isobutyronitril und eines inerten organischen Verdünnungsmittels mit Tri-n-butylzinnhydrid oder Tri-n-propylsilan behandelt wird, um 3-Acetyl-13-deoxy-$GA_3$ zu bilden, und das 3-Acetyl-13-deoxy-$GA_3$ mit Alkali behandelt wird, um das 3$\beta$-Acetyl-13-deoxy-$GA_3$ unter Bildung von $GA_7$ zu deacetylieren.

12. Verbindung der Formel

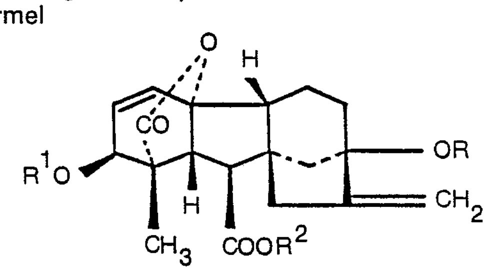

worin R für unsubstituiertes oder halogen-substituiertes Alkansulfonyl, unsubstituiertes oder halogen-, alkyl- oder trifluoromethyl-substituiertes Benzolsulfonyl oder unsubstituiertes oder halogen-, alkyl- oder trifluoromethyl-substituiertes Benzolcarbonyl oder -thiocarbonyl steht, $R^1$ für Wasserstoff oder Acyl (vorzugsweise Alkanoyl, z.B. Acetyl) steht und $R^2$ für Wasserstoff, Silyl oder unsubstituiertes oder halogen-, nitro-, alkyl-, alkoxy- oder trifluoromethyl-substituiertes Phenacyl steht.

13. Verbindung nach Anspruch 12, dadurch gekennzeichnet, daß R für Methansulfonyl, p-Toluolsulfonyl, Benzolsulfonyl oder Benzoyl steht.

14. 3-Acetyl-13-mesyloxy-$GA_3$.

15. Eine Verbindung nach Anspruch 12 in Form eines Säurehalogenids (z.B. Säurechlorids).

**Revendications**

1. Procédé de 13-déshydroxylation d'une 13-hydroxygibberelline, dont le groupe 7-carboxy est éventuellement protégé, et la 13-hydroxygibberelline contient un groupe hydroxyallylique et un pont 19,10-lactone, caractérisé par la transformation du groupe 13-hydroxy, éventuellement sous la forme d'un sel de métal alcalin, de la 13-hydroxygibberelline en un ester phosphorique, sulfonique, carboxylique, thiocarboxylique ou dithiocarbonique, puis la réduction du 13-ester ainsi formé en présence d'un initiateur de radicaux libres et d'un hydrure organique d'étain ou d'un organosilane pour réduire le 13-ester et pour former une gibberelline 13-déshydroxylée, qui est amenée à réagir, le cas échéant, d'une manière connue pour convertir le groupe protégeant la position 7 en un groupe 7-carboxy.

2. Procédé suivant la revendication 1, caractérisé en ce que le 13-ester est formé par réaction de la 13-hydroxygibberelline avec un agent estérifiant qui est un acide phosphorique, un acide sulfonique ou un acide carboxylique ou un dérivé estérifiant de l'un desdits acides, par exemple un halogénure de (alcane non substitué ou à substituant halogéno)-sulfonyle, un halogénure de (arène non substitué ou à substituant halogéno, nitro, alkyle, alkoxy ou trifluorométhyle)-sulfonyle ou un halogénure d'acide (arène non substitué ou à substituant halogéno, nitro, alkyle, alkoxy ou trifluorométhyle)-carboxylique.

3. Procédé suivant la revendication 2, caractérisé en ce que l'agent estérifiant est un chlorure d'alcane-sulfonyle non substitué, par exemple le chlorure de méthane- ou d'éthane-sulfonyle, un chlorure d'arène-sulfonyle non substitué ou à substituant alkyle, par exemple le chlorure de p-toluènesulfonyle ou de benzènesulfonyle ou un chlorure d'acide arène-carboxylique non substitué, par exemple le chlorure d'acide benzoïque.

4. Procédé suivant l'une quelconque des revendications 1 à 3, caractérisé en ce que l'hydrure organique d'étain ou l'organosilane répond à la formule

$$R_xMH_y$$

dans laquelle M représente Sn ou Si, R est un groupe alkyle, aryle ou aralkyle non substitué et $x$ et $y$ sont égaux à 1, 2 ou 3, la somme de $x$ et $y$ étant égale à 4; et, lorsque $x$ est égal à 2 ou 3, les groupes R sont égaux ou différents.

5. Procédé suivant la revendication 4, caractérisé en ce que, dans l'hydrure organique d'étain ou l'organosilane, R est un groupe alkyle en $C_1$ à $C_6$, phényle ou benzyle, $x$ est égal à 3 et $y$ est égal à 1.

6. Procédé suivant la revendication 5, caractérisé en ce que l'hydrure organique d'étain est l'hydrure de tri-n-butylétain, ou bien l'organosilane est le tri-n-propylsilane.

7. Procédé suivant l'une quelconque des revendications 1 à 6, caractérisé en ce que la 13-hydroxygibberelline contient au moins un autre groupe hydroxy.

8. Procédé suivant la revendication 7, caractérisé en ce que l'autre groupe hydroxy est protégé avant l'estérification du groupe 13-hydroxy, et le groupe protecteur est éliminé avant, après et/ou pendant la déshydroxylation en position 13.

9. Procédé suivant l'une quelconque des revendications 1 à 8, caractérisé en ce que le groupe protégé en position 7 de la 13-hydroxygibberelline est un ester de phénacyle non substitué ou à substituant halogéno, nitro, alkyle, alkoxy ou trifluorométhyle ou un ester de silyle.

10. Procédé suivant la revendication 9, caractérisé en ce que la 13-hydroxygibberelline est $GA_3$ et la gibberelline déshydroxylée en position 13 est $GA_7$.

11. Procédé suivant la revendication 10, caractérisé en ce que la gibberelline $GA_3$ est acétylée pour former la 3-acétyl-$GA_3$, la 3-acétyl-$GA_3$ est traitée avec le chlorure de mésyle pour former la 3-acétyl-13-mésyloxy-$GA_3$, la 3-acétyl-13-mésyloxy-$GA_3$ est traitée avec l'hydrure de tri-n-butylétain ou le tri-n-propylsilane en présence d'$\alpha$-azo-isobutyronitrile et d'un diluant organique inerte pour former la 3-acétyl-13-déoxy-$GA_3$ et la 3-acétyl-13-déoxy-$GA_3$ est traitée avec un alcali pour désacétyler la 3$\beta$-acétyl-13-déoxy-$GA_3$ de manière à former la $GA_7$.

12. Composé de formule:

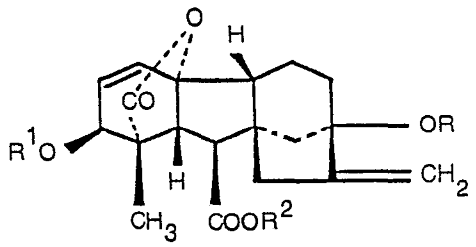

dans laquelle R est un groupe (alcane non substitué ou à substituant halogéno)-sulfonyle, (benzène non substitué ou à substituant halogéno, alkyle ou trifluorométhyle)sulfonyle ou (benzène non substitué ou à substituant halogéno, alkyle ou trifluorométhyle)-carbonyle ou -thiocarbonyle, $R^1$ est l'hydrogène ou un groupe acyle (de préférence un groupe alcanoyle tel que le groupe acétyle) et $R^2$ est l'hydrogène, le groupe silyle ou un groupe phénacyle non substitué ou à substituant halogéno, nitro, alkyle, alkoxy ou trifluorométhyle.

13. Composé suivant la revendication 12, caractérisé en ce que R est un groupe méthanesulfonyle, p-toluènesulfonyle, benzènesulfonyle ou benzoyle.

14. La 3-acétyl-13-mésyloxy-$GA_3$.

15. Composé suivant la revendication 12, sous la forme d'un halogénure d'acide (par exemple le chlorure d'acide).